Europäisches Patentamt

European Patent Office

Office européen des brevets

(11) Publication number: 0 428 914 A1

# EUROPEAN PATENT APPLICATION

(21) Application number: 90120876.9

(22) Date of filing: 31.10.90

(51) Int. Cl.⁵: A61B 5/14

(30) Priority: 13.11.89 US 435985

(43) Date of publication of application:
29.05.91 Bulletin 91/22

(84) Designated Contracting States:
DE FR GB

(71) Applicant: MILES INC.
1127 Myrtle Street
Elkhart,IN 46515(US)

(72) Inventor: Brenneman, Allen J.
24479 C.R. 26
Elkhart, Indiana 46517(US)
Inventor: Brown, Michael K.
56123 Neely Court
Elkhart, Indiana 46516(US)

Inventor: Purcell, D. Glenn
70953 Brande Creek Drive
Edwardsburg, Michigan 49112(US)
Inventor: Taylor, William A.
60652 C.R. 27
Goshen, Indiana 46526(US)
Inventor: Whitson, Robert C.
10663 E. Jefferson Road
Osceola, Indiana 46561(US)
Inventor: Zuidema,Jack L.
53713 C.R. 11 North
Elkhart, Indiana 46514(US)

(74) Representative: Kirchner, Dietrich, Dr. et al
c/o BAYER AG Konzernverwaltung RP
Patentabteilung
W-5090 Leverkusen, Bayerwerk(DE)

(54) Lancet assembly.

(57) A lancet assembly (10, 100) for making a puncture in a finger includes a housing (12, 102) fabricated of flexible, disposable material. A spring (22) including a first end (30) defining a lancet (32) is mounted in the housing in a stressed position. Upon release of the spring the lancet passes through an opening (34) in the housing to strike a finger positioned against the housing. The lancet (32) is then returned to within the housing by the natural resiliency of the spring (22). In a second embodiment of the lancet assembly (100) the housing (102) is reusable, and a disposable lancet cartridge (104) is mounted on the housing. A cantilever spring (132) is mounted in the housing and is cocked by a slide (146, 148) on the housing. Upon being released from the cocked position, the spring (132, 136) strikes a hammer member (138) engaging an end (112) of a lancet (114) in the lancet cartridge. After puncturing a finger, the lancet is returned to a position with in the cartridge by return springs (130). The cartridge is then removed from the lancet assembly and discarded.

## FIG. 6

## LANCET ASSEMBLY

### BACKGROUND OF THE INVENTION

#### A. Field of the Invention

The device of the present invention generally relates to a new and improved lancet assembly for puncturing the skin of a user to obtain a blood sample; and, more particularly, to a new and improved disposable lancet assembly including a minimum number of parts.

#### B. Description of the Prior Art

Sharp pointed lancets are employed to make a puncture or penetration of a patient's skin in order to provide a small outflow of blood. Since various tests may be employed with only small amounts of blood, blood flowing from a finger puncture is normally sufficient for these tests. Tests on the blood often include contacting a paper strip or reagent pad on a strip carrying chemistry with the blood from the puncture or wound.

Lancet assemblies now available include a driving member that grips a lancet. Loading and removing a lancet from the driving member of the assembly requires extra care by the user to avoid being punctured, since the lancet must be carefully gripped to avoid contact with the sharp end of the lancet. Present day concerns about communicable diseases transmitted through body fluids such as blood increase the risks of handling these lancets. Current devices require the user to remove a protective cover from the sharp end of a lancet, and load the exposed lancet into a lancet assembly. After use of the assembly, the point of the lancet is exposed. Users of these devices, such as nurses, are repeatedly handling these used, exposed lancets and have a high risk of puncture and resultant exposure to contamination through the blood on the lancet.

Some designs of lancets include a end cap with the sharp end of the lancet positioned within the end cap after use, and the opposite end of the lancet sticking out of the end cap. Upon removal of the end cap after use, it is possible for the user to be stuck with an infected lancet simply by pressing on the exposed end of the lancet thereby moving the sharp end of the lancet out of the end cap. This risk is especially high for professional users such as nurses who place used lancets in their pockets. At the end of the day these nurses reach into their pockets and pull out used items including lancets. Upon reaching into his or her pocket, the nurse can press the back end of a lancet thereby exposing the contaminated sharp point.

One example of a known lancet is illustrated in U.S. Patent No. 3,030,959. The lancet of this patent includes a spindle designed for housing a replaceable magazine containing a stock of needles. A feeding device for feeding a needle to be used is also disclosed. After a needle is used, it is maintained in the lancet until another needle from the magazine is loaded. As the new needle is advanced, the needle preceding it is ejected from the lancet. The lancet assembly of U.S. Patent No. 3,030,959 ejects an unprotected, exposed and contaminated needle which increases the risk of puncture by the user of the lancet assembly.

A disposable needle probe package is disclosed in U.S. Patent No. 4,637,403. The needle package is frictionally held in the medical testing system by snapping, threading or similar procedures, and it is releasable by pushing the probe package out of the medical monitoring system. Once the probe package is released from the monitoring system, however, the needle is exposed and handling can result in puncturing the skin of the user thereby exposing the user to contaminated blood.

A lancet assembly intended to be disposable with no part of the assembly reused is disclosed in U.S. Patent No. 4,375,815. Examples of other lancet assemblies intended to be totally disposable, as opposed to disposing only the lancet, are disclosed in U.S. Patent Nos. 4,388,925; 4,553,541; 4,449,529 and 4,535,769. These lancet assemblies include multiple parts which result in increased cost per lancet assembly.

A disposable lancet defined by a plunger is disclosed in United States Patent Nos. 4,712,548 and 4,738,261. After this device has been used, the lancet is exposed with the risk that someone handling the used device could be punctured by the contaminated lancet.

Lancet assemblies in which used, exposed lancets must be removed with the risk of puncture are disclosed in United States Patent Nos. 4,416,279; 4,462,405; 4,442,836 and 4,469,110.

United States Patent No. 4,545,376 discloses a one piece, plastic lancet consisting of a handle and a tip. Once a protective yoke is removed, the lancet tip is completely exposed and can accidentally puncture a user.

Lancet assemblies that are totally disposable with no reusable components are disclosed in United States Patent Nos. 4,624,253; 4,616,649 and 4,539,988.

A lancet assembly with a lancet exposed after use is disclosed in United States Patent No. 4,452,243.

There is a need for a lancet assembly in which the lancet is completely contained within a portion of the lancet assembly both before and after use such that the user can dispose of the lancet without risk of puncture. Such a lancet assembly preferably includes a minimum number of components to ensure the lowest possible cost per assembly.

## SUMMARY OF THE INVENTION

Briefly, the present invention is directed to a new and improved device commonly referred to as a lancet assembly used for puncturing the skin of a user or a patient to allow blood from the puncture to be drawn and tested. A first embodiment of the lancet assembly includes a housing made of flexible resilient material such as plastic. A spring is mounted within the housing and held by a holding member in the stressed or stored energy position. The spring includes a first end secured to the housing and a second end that is formed to define a skin piercing end or a lancet.

In the stressed position the second end of the spring is located above an opening in the housing. To activate the lancet, one side of the housing is flexed to move the holding member out of engagement with the spring. Once released, the second end of the spring moves downward toward the opening in the housing. An abutment member is formed on the housing adjacent to the opening. The abutment member is engaged by the spring and imparts a whipping motion to the second end of the spring as it passes through the opening and into the finger of a user or patient. After puncturing the skin of the user or patient, the resiliency of the spring returns it to a position within the housing abutting the abutment member. In this position, the second end of the spring is totally within the housing, and the lancet assembly can be safely handled without the danger of puncturing the individual who is carrying or disposing of the lancet assembly.

A second embodiment of the lancet assembly includes a housing within which is mounted a spring. A disposable lancet cartridge including a lancet is slideably mounted on the housing. One end of the lancet is adjacent to an opening in the housing in a position to be struck by the spring. The spring is cocked to a stressed or stored energy position by a cocking mechanism that moves a free end of the spring into holding engagement with a spring holder. The spring holder is actuated to release the spring. The lancet is then driven through an opening in the lancet cartridge to pierce a finger positioned against the lancet cartridge. A spring return mechanism is included within the lancet cartridge to return the lancet to a position within the lancet cartridge such that the user can then eject the cartridge and dispose of it without being punctured by the contaminated lancet.

## BRIEF DESCRIPTION OF THE DRAWINGS

The above and other advantages and novel features of the present invention will become apparent upon reading the following detailed description of a preferred embodiment of the invention illustrated in the accompanying drawings wherein:

FIG. 1 is a partially cut-away, side view of a lancet assembly constructed in accordance with the principles of the present invention with the spring in the stressed or stored energy position;

FIG. 2 is a view of the lancet assembly of the present invention taken generally along line 2-2 in FIG. 1;

FIG. 3 is a side view of the lancet assembly similar to FIG. 1 illustrating the spring in the skin piercing position;

FIG. 4 is a view similar to FIG. 3 illustrating the spring in the equilibrium or stable position after piercing a finger;

FIG. 5 is a perspective view of an alternative embodiment of the lancet assembly of the present invention;

FIG. 6 is a view taken generally along line 6-6 in FIG. 5.

FIG. 7 is a partially broken-away, bottom plan view of the lancet assembly illustrated in FIG. 5 with the lancet cartridge removed; and

FIG. 8 is an enlarged view taken generally along line 8-8 of FIG. 5.

## DESCRIPTION OF THE PREFERRED EMBODIMENTS

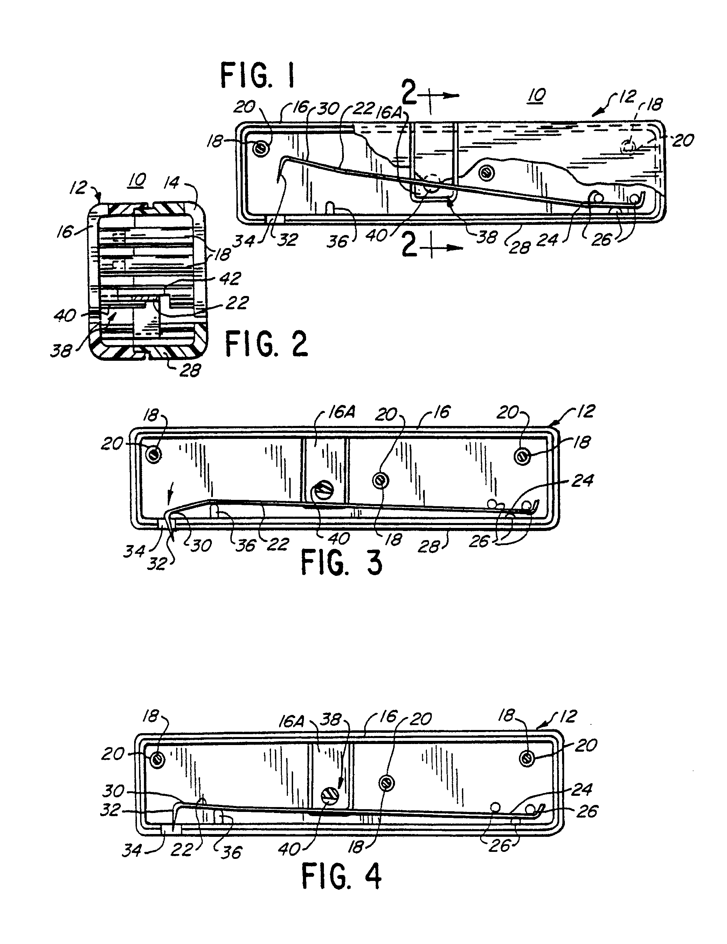

Referring to the drawings and initially to FIGS. 1-4, there is illustrated a lancet device or assembly generally designated by the reference numeral 10. The lancet assembly 10 includes a housing 12 defined by a first housing portion 14 and a second housing portion 16. For ease of assembly, several snap pins 18 are formed on housing portion 14 which are snapped into bosses 20 formed on the second housing portion 16 to hold the housing portions 14 and 16 together and form housing 12.

To minimize the number of parts required in the lancet assembly 10 a multi-functioned flat metal cantilever spring 22 is mounted within the housing 10 at a first end 24 by three mounting lugs 26 formed on the second housing portion 16 adjacent a bottom wall 28 of the housing 12. It is to be understood that it lu is not intended to limit spring 22 to a flat metal. The spring 22 could also be a round wire, a conical spring or any other spring.

The spring 22 includes a second end 30 that is ground to form a sharp point or lancet 32.

The multi-purpose spring 22, when released, drives the lancet 32 downwardly and through an opening 34 in the bottom wall 28 to pierce a finger that has been pressed against the opening 34. By placing the first end 24 of the spring 22 adjacent the bottom wall 28, the lancet or sharpened second end 32 passes through the opening 34 along a more linear or straight path providing a cleaner, less painful puncture in the finger of a user or patient.

To ensure a quick puncture and quick removal of the lancet 32 from the finger of a patient or user, an abutment or stop bump 36 is formed on the bottom wall 28 of the housing 12 near the opening 34. As illustrated in FIG. 3, after release of the spring 22 from its stressed or stored energy position, the spring 22 engages the stop bump 36 which causes a whipping action of the lancet 32. The stop bump 36 also assists in quickly returning the lancet 32 to a position within the housing 12 as best illustrated in FIG. 4. In the equilibrium or stable position illustrated in FIG. 4, the lancet 32 is completely within the housing 12. The lancet assembly 10 can then be handled by a patient, a user or a nurse without danger of being pierced by the contaminated lancet 32.

The spring 22 is held in the stressed or stored energy position by a spring retainer generally designated by the reference numeral 38. The spring retainer 38 includes a spring holder 40 formed on a portion 16A of the wall 16. The portion 16A of the wall 16 is defined by a cut along three sides of the portion 16A. This cut allows the portion 16A to flex relative to the second housing portion 16, The spring retainer 38 also includes a peg or finger 42 (Fig. 2) formed on the first housing portion 14. The spring 24 is released by squeezing on the first housing portion 14 in the general area of the peg or finger 42. This area of the first housing portion 14 may have a cut out portion similar to the portion 16A. This causes the peg or finger 42 to move against the wall portion 16A flexing it outwardly and moving the spring holder 40 from beneath the spring 22. Once the spring holder 40 is completely removed from beneath the spring 22, the spring 22 is released to move downwardly engaging the stop bump 36 and whipping the lancet 32 into and through the opening 34 to pierce a user's or patient's finger. The spring 22 is then returned to the stable or equilibrium position illustrated in FIG. 4. Once used in this manner, the lancing device or lancet assembly 10 may be discarded.

The lancet assembly 10 requires only two parts, the housing 12 and the spring 22. The spring 22 is multi-functioned whereas prior lancet assemblies typically include at least a spring to drive a lancet and a second spring to return the lancet to a position Within the housing of the device. The spring 22 of the present invention provides both the driving force and the returning force, thus minimizing the number of parts required and significantly reducing the cost. In addition, the lancet assembly 10 is easy to use requiring only one step; squeezing the housing 12 to actuate the lancet 32 and to pierce the finger of a user or patient. After use, the lancet assembly 10 is simply disposed of without the necessity of precautionary steps.

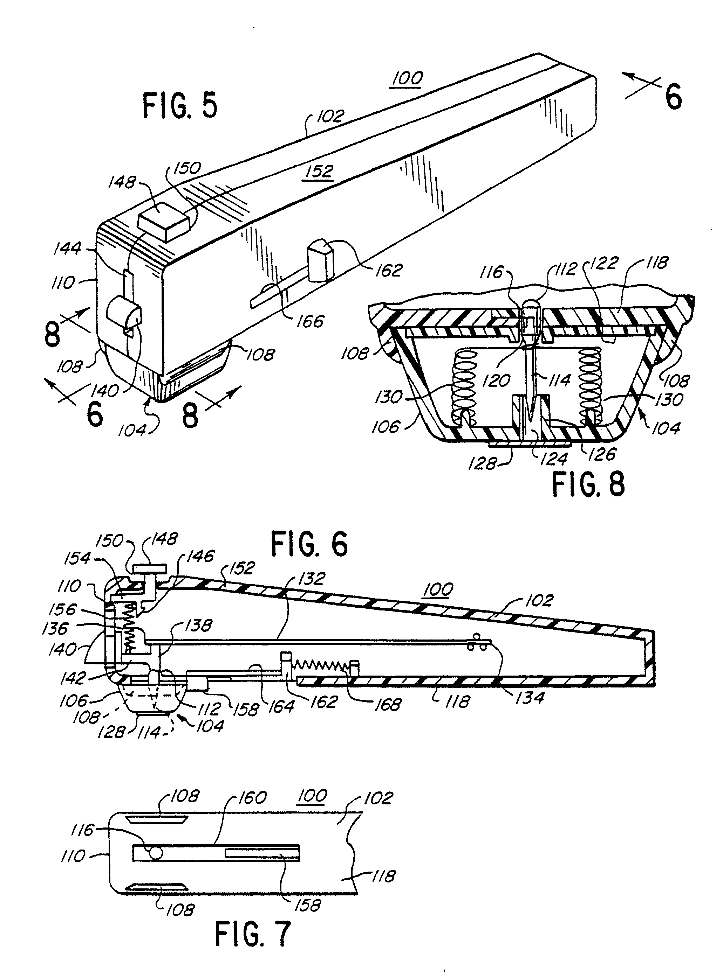

Referring now to FIGS. 5-7 there is illustrated an alternative lancet assembly 100. The lancet assembly 100 includes two components; a reusable body 102 and a disposable lancet cartridge 104. The lancet cartridge 104 includes a housing 106 constructed of inexpensive, disposable material such as plastic. The sides of the housing lu6 are tapered to fit between a pair of rails 108 formed on the forward end llu of the housing 102. The disposable lancet cartridge 104 is mounted on the lancet assembly 100 by sliding it between the rails 108 until a head 112 of a lancet 114 is located within an aperture 116 defined in a bottom wall 118 of the body 102. The disposable lancet cartridge 104 includes the lancet 114 aligned with an upper aperture 120 in an upper wall 122 of the housing 106. Once the lancet cartridge 104 is mounted on the body 102, the head 112 of the lancet 114 extends through the aperture 120.

The lancet 114 is mounted in the housing 106 in a position within an aperture 124 formed by a circular wall 126. The aperture 124 is covered by a seal 128 that is a material that can be perforated by the lancet 114, but which protects the lancet 114 from contamination prior to use. The lancet 114 is biased by springs 130 to a position totally within the housing 106 to avoid accidental puncture by the user. The springs 130 also function to return the lancet 114 to the position illustrated in FIG. 8 after the lancet 114 has been driven into the finger of a user or patient. By returning the lancet 114 to a position within the housing 106, the lancet cartridge 104 can be removed and handled by a nurse or user without danger of accidental puncture by the contaminated lancet 114.

The lancet 114 is driven out of the housing 106 and into the finger of a user or patient by a cantilever spring 132 that is rigidly secured to the body 102 at a first end 134 of the spring 132. To drive the lancet 114, a second end 136 of the cantilever spring 132 strikes a hammer member 138 resting on the head 112 of the lancet 114. Prior to driving the lancet 114, the cantilever spring 132 is raised to or cocked and held in an elevated position. This is accomplished by moving the hammer member 138 upwardly with the second end 136 of the cantilever spring 132. The upward movement of the hammer member 13s is accomplished by moving a button 140 on the front end 110 of the housing 102. The button 140 is connected to the hammer member 138 by a connecting beam 142. To cock the cantilever spring 132 a nurse or user places a finger below and in engagement with the button 140 and moves the button upwardly in a slot 144 until the second or free end 136 of the cantilever spring 132 engages and is held by a hook 146 formed on a plunger 148. The plunger 148 extends through an aperture 150 in an upper wall 152 of the body 102. The button 142 is mounted by a leg 154 rigidly secured in the front end 110 of the body 102. Once the second end 136 of the cantilever spring 132 is raised and held by the hook 146, the button 140 is released and a spring 156 moves the button 140 downward to the bottom of the slot 144. In this positions the hammer member 138 is in engagement with the head 112 of the lancet 114.

The driving of the lancet 114 is commenced by the user of the lancet assembly 100 gripping the body 102 and depressing the plunger 148 using a thumb or finger. As the plunger 148 is depressed it pivots about the leg 154 at the point where the leg 154 is secured to the front end 110 of the body 102. This pivoting moves the hook 146 in an arc out of engagement with the second or free end 136 of the cantilever spring 132. The cantilever spring 132 is released and snaps downwardly to strike the hammer member 138 driving the lancet 114 through the aperture 124. After striking the hammer member 138, the spring 132 returns to its normal, at rest position best illustrated in FIG. 6. The springs 130 return the lancet 114 completely within the housing 106 of the disposable lancet cartridge 104.

Once this has been accomplished, the user can eject the lancet cartridge 104 from the housing 102 by an ejection finger 158. The ejection finger 158 slides within a slot 160 formed in the bottom wall 118 of the body 102. As the ejection finger 158 engages the housing 106 of the disposable lancet cartridge 104, the disposable lancet cartridge 104 is moved forwardly along and eventually off the rails 108. The user may then dispose of the lancet cartridge 104 in a safe location.

The ejection finger 158 is connected to a slide member 162 by an arm 164. The slide 162 extends through a slot 166 formed in the body 102. The slide 162 may be engaged by a thumb or finger of the user and moved forwardly within the slot 166 to engage the ejection finger 158 with the disposable lancet cartridge 104. When the disposable lancet cartridge 104 has been removed from the lancet assembly 100, the slide 162 can be released and it will be returned to its original position as illustrated in FIG. 5 under the influence of a spring 168. The ejection finger 158 is then moved into a position such that a new disposable lancet cartridge 104 can be mounted on the body 102.

The lancet assembly 100 provides a reusable device with the safety of disposable lancet cartridges 104. The cartridges 104 can be used and disposed of without the risk of an accidental or inadvertent puncture by the lancet 114 before it has been used or when it is contaminated after use. In addition, through the use of the cantilever spring 132 the lancet assembly 100 is compact, has a minimum number of parts, and due to the reduced manufacturing and materials costs, is inexpensive to the user.

## Claims

1. A disposable lancing device, comprising a housing, and a spring mounted in said housing for driving a lancet outside said housing and returning with said lancet to an equilibrium position inside said housing, said spring including a first end secured in said housing, and a second end releasably held by said housing in a stored energy position, said second end of said spring defining said lancet.

2. The disposable lancing device set forth in claim 1 wherein said housing includes a holding member for releasably holding said second end of said spring in said stored energy position, said housing further including a flexible portion, said holding member formed on said flexible portion of said housing so as to release said second end of said spring upon flexing of said flexible portion.

3. The disposable lancing device set forth in claim 1 further comprising a spring engagement member in said housing for engagement by said spring upon said spring being released to impart a whipping motion to said spring and to hold said spring inside said housing in the equilibrium position of said spring.

4. The disposable lancing device set forth in claim 1 wherein said spring is a cantilever spring.

5. The disposable lancing device set forth in claim 1 wherein said housing includes a bottom wall, an opening in said bottom wall through which said second end of said spring extends to pierce a finger, a mounting element in said housing for mounting said first end of said spring adjacent said bottom wall.

6. A disposable, single use lancing device for piercing a finger with a lancet, for removing said lancet from said finger, and for holding said lancet safely inside said lancing device after piercing said finger, comprising: a housing,

a spring mounted in a stored energy position in said housing, said spring including a first end formed to define a lancet, and

means for releasing said spring to drive said lancet out of said housing to pierce a finger whereupon the resiliency of said spring returns said spring to an equilibrium position with said lancet in said housing.

7. A disposable, single use, safe lancing device, comprising:

a housing formed of a resilient material, said housing including at least one wall, said wall formed to be flexible, a mounting member provided on the inner surface of said flexible wall;

a metal spring, mounted in said housing n a stored energy position, said metal spring including a first end secured to said housing, said spring including a second, sharp end mounted on said mounting member for release upon flexing of said flexible wall, and

means for maintaining said second, sharp end of said spring completely within said housing after release of said spring and return of said spring to an equilibrium position.

8. A blood lancet device comprising:

a housing;

a lancet; and

a cantilever spring mounted in said housing for driving said lancet to pierce a finger.

9. The blood lancet device claimed in claim 8 wherein said lancet comprises a disposable lancet cartridge removably mounted on said housing, said disposable lancet cartridge including a lancet with a first end adapted to be struck by said cantilever spring to drive said lancet.

10. The blood lancet device set forth in claim 8 wherein said lancet comprises a lancet cartridge slideably mounted on said housing, said lancet cartridge including a lancet and a return spring for returning said lancet to within said cartridge after being driven a hammer member in said housing for driving said lancet upon being struck by said cantilever spring, and a holding element in said housing for holding said cantilever spring in a stored energy position.

FIG. 1

FIG. 2

FIG. 3

FIG. 4

FIG. 5

FIG. 8

FIG. 6

FIG. 7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,A | GB-A-2 098 486 (RYDER INT. CORP.)<br>* page 1, lines 4 - 6 * * page 2, lines 4 - 68; figures 1-5 *<br>– – – | 1-5,8,7 | A 61 B<br>5/14 |
| X,A | US-A-3 760 809 (S.P. CAMPBELL, JR.)<br>* column 1, lines 4 - 39; figures * * column 2, line 40 - column 5, line 62 *<br>– – – | 1-5,8,7 | |
| X | EP-A-0 097 748 (G.J. SLAMA)<br>* page 4, line 7 - page 6, line 11 * * figures 1-4 *<br>– – – | 1,2,5-7 | |
| X,A | US-A-4 577 630 (R.P. NITZSCHE + K. E. GEIGER)<br>* column 7, line 50 - column 8, line 47 * * figures 1, 2, 10-13 *<br>– – – | 6,8,9 | |
| A | DE-A-2 131 297 (R. PHILIPP)<br>* page 2, line 8 - page 3, line 21; figures * * page 4, line 16 - page 6, line 21 *<br>– – – – – | 1,5,7-10 | |

|  | TECHNICAL FIELDS SEARCHED (Int. Cl.5) |
|---|---|
|  | A 61 B |

The present search report has been drawn up for all claims

| Place of search | Date of completion of search | Examiner |
|---|---|---|
| The Hague | 14 February 91 | RIEB K.D. |